(19) Europäisches Patentamt
European Patent Office
Office européen des brevets

(11) Publication number: **0 365 139 A2**

(12) # EUROPEAN PATENT APPLICATION

(21) Application number: 89309374.0

(51) Int. Cl.[5]: **A61K 31/575**

(22) Date of filing: 14.09.89

The title of the invention has been amended (Guidelines for Examination in the EPO, A-III, 7.3).

(30) Priority: 20.09.88 GB 8822012
20.09.88 GB 8822015
20.09.88 GB 8822020

(43) Date of publication of application:
25.04.90 Bulletin 90/17

(84) Designated Contracting States:
ES GR

(71) Applicant: **FISONS plc**
**Fison House Princes Street**
**Ipswich Suffolk IP1 1QH(GB)**

(72) Inventor: **Sharpe, Christopher James**
**126 Ellesmere Road**
**Shrewsbury Shropshire, SY1 2QZ(GB)**

(74) Representative: **Craig, Christopher Bradberry et al**
**Fisons plc 12 Derby Road**
**Loughborough Leicestershire LE11 0BB(GB)**

(54) **Bile acids for the treatment of viral infections.**

(57) Compounds of formula I,

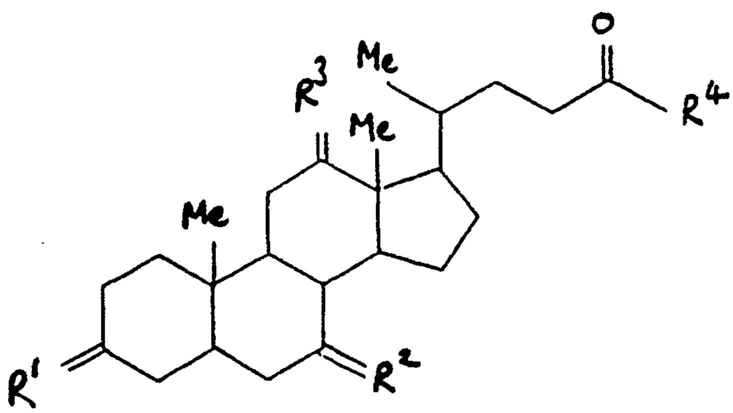

I

wherein
$R^1$ and $R^2$ both represent (H,α-OH) or both represent O;
$R^3$ represents (H,H) or O; and
$R^4$ represents OH or $NH(CH_2)_2SO_3H$;
and pharmaceutically acceptable salts thereof;
provided that when $R^1$ and $R^2$ both represent (H,α-OH) and $R^3$ represents (H,H), then $R^4$ represents $NH(CH_2)_2SO_3H$;
are useful as pharmaceuticals, in particular in the treatment of diseases caused by enveloped viruses.



Xerox Copy Centre

## METHOD AND COMPOSITION FOR THE TREATMENT OF VIRAL INFECTIONS

This invention relates to a new use of certain known compounds in the treatment of viral infections caused by enveloped viruses, and to pharmaceutical compositions containing them.

Naturally occuring bile acids are produced by the liver, and certain of these have been used in the treatment of gallstones for a number of years. More recently, European Patent Application 0285285 disclosed the use of bile acids in the treatment of viral infections.

We have now surprisingly found a small group of bile acids and pharmaceutically acceptable salts thereof which are particularly useful in the treatment of diseases caused by enveloped viruses.

Thus, according to the present invention, there is provided a compound of formula I,

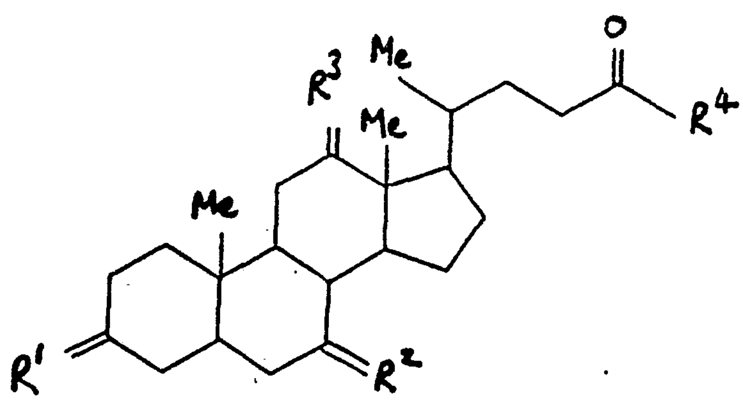

I

wherein

$R^1$ and $R^2$ both represent (H,$\alpha$-OH) or both represent O;

$R^3$ represents (H,H) or O; and

$R^4$ represents OH or $NH(CH_2)_2SO_3H$;

and pharmaceutically acceptable salts thereof;

provided that

i) when $R^1$ and $R^2$ both represent (H,$\alpha$-OH) and $R^3$ represents (H,H), then $R^4$ represents $NH(CH_2)_2SO_3H$; and

ii) when $R^1$ and $R^2$ both represent O and $R^3$ represents O, then $R^4$ represents $NH(CH_2)_2SO_3H$; for use as a pharmaceutical.

We further provide the use of a compound of formula I as defined above but without proviso (ii), as active ingredient in the manufacture of a medicament for the treatment or prophylaxis of diseases caused by enveloped viruses.

The compounds which we provide as pharmaceuticals are also known as 3,7-diketocholanic acid [$R^1$ and $R^2$ both represent O, $R^3$ represents (H,H) and $R^4$ represents OH]; tauro-3,7-diketocholanic acid [$R^1$ and $R^2$ both represent O, $R^3$ represents (H,H) and $R^4$ represents $NH(CH_2)_2SO_3H$]; taurodehydrocholic acid [$R^1$ and $R^2$ both represent O, $R^3$ represents O and $R^4$ represents $NH(CH_2)_2SO_3H$]; taurochenodeoxycholic acid [$R^1$ and $R^2$ both represent (H,$\alpha$-OH), $R^3$ represents (H,H) and $R^4$ represents $NH(CH_2)_2SO_3H$];

3$\alpha$,7$\alpha$-dihydroxy-12-ketocholanic acid [$R^1$ and $R^2$ both represent (H,$\alpha$-OH), $R^3$ represents O and $R^4$ represents OH]; and

tauro-3$\alpha$,7$\alpha$-dihydroxy-12-ketocholanic acid [$R^1$ and $R^2$ both represent (H,$\alpha$-OH), $R^3$ represents O and $R^4$ represents $NH(CH_2)_2SO_3H$].

The compound which we provide for use in the manufacture of a medicament for the treatment or prophylaxis of diseases caused by enveloped viruses in addition to those named above is known as dehydrocholic acid [$R^1$ and $R^2$ both represent O, $R^3$ represents O and $R^4$ represents OH].

Tauro derivatives may be obtained from the parent bile acid by a mixed anhydride synthesis, eg using ethyl chloroformate and taurine.

At physiological pH, bile acids are usually ionised and the terms 'bile acid' and 'bile salt' are used interchangeably. Pharmaceutically acceptable salts of the compounds which may be mentioned include certain alkali metal salts, particularly the sodium salts, and certain alkaline earth metal salts, particularly the calcium salts.

By "enveloped virus" we mean a virus having a lipid outer coat. Included in this definition are the

human immunodeficiency, influenza, parainfluenza and herpes viruses. Thus, diseases of particular interest are AIDS, influenza, parainfluenza and herpes.

We prefer the compound of formula I as defined above to be dehydrocholic acid or taurochenodeoxycholic acid.

The compound of formula I may be administered to a patient by any convenient means which results in their introduction into the systemic circulation. Thus they may be introduced parenterally eg by injection (intravenously, intramuscularly or subcutaneously), topically, orally, using plasmaphoresis, or by inhalation in the form of a non-pressurised powder or an aerosol formulation.

Thus, the invention further provides a pharmaceutical composition comprising as active ingredient at least one compound of formula I as defined above (preferably less than 80%, and more preferably less than 50% by weight) in admixture with a pharmaceutically acceptable adjuvant, diluent or carrier.

Examples of suitable adjuvants, diluents or carriers are: for tablets, capsules and dragees - microcrystalline cellulose, calcium phosphate, diatomaceous earth, a sugar such as lactose, dextrose or mannitol, talc, stearic acid, starch, sodium bicarbonate and/or gelatin; for suppositories - natural or hardened oils or waxes; and for inhalation compositions - coarse lactose. The compound of formula I is preferably in a form having a mass median diameter of from 0.01 to 10 microns. The compositions may also contain suitable preserving, stabilising and wetting agents, solubilisers, sweetening and colouring agents and flavourings. The compositions may, if desired, be formulated in sustained release form.

For the above-mentioned therapeutic uses the dosage administered will vary with the compound employed, the mode of administration and the disease indicated. However, in general, satisfactory results are obtained when the compounds are administered at a dosage which produces a concentration in the blood stream of from 1-100μg/ml, preferably 5-100μg/ml.

For man the indicated total daily dosage is in the range of from 1mg to 2500mg and preferably from 10mg to 2000mg, which may be administered, for example twice weekly, or in divided doses from 1 to 6 times a day or in sustained release form. Thus unit dosage forms suitable for administration, eg oesophageally, comprise from 1mg to 2500mg, and preferably 2mg to 2000mg of the compound preferably admixed with a solid or liquid pharmaceutically acceptable diluent, carrier or adjuvant.

According to a further aspect of the present invention there is provided a method of treatment or prophylaxis of a disease caused by an enveloped virus, which comprises administration of a therapeutically effective amount of a compound of formula I as defined above to a patient suffering from or at risk of contracting such a disease.

The compounds of formula I have the advantage that they are less toxic, more efficacious, are longer acting, have a broader range of activity, are more potent, produce fewer side effects, are more easily absorbed or have other useful pharmacological properties, than compounds previously used against the diseases mentioned above.

The antiviral activity of the compounds of formula I which may be used in the present invention were tested in the procedures set out in Tests A and B below.

## Test A

Mammalian lymphocyte (H9IIIB) cells which were chronically infected with HIV were diluted to a concentration of $2x10^5 ml^{-1}$ in a culture medium (RPMI 1640 - which is a mixture of amino acids, salts, glucose and vitamins available from Gibco). 0.5ml aliquots of this solution were placed in the wells of a tissue culture plate together with 0.5ml aliquots of an aqueous solution of the compound under test. A range of initial concentrations of test compound solution were used (0.4, 4, 40 and $400 mgml^{-1}$), thus giving final test compound concentrations of 0.1, 1, 10 and $100 mgml^{-1}$ in the wells.

Uninfected human lymphocyte (8166) cells (1ml of a $1x10^6 ml^{-1}$ solution in a culture medium) were then added to some wells, and culture medium alone (1ml) was added to the remaining wells.

The infected cell/test compound mixtures were then incubated at 37° C.

On following days, for example day 1 and day 4, 200μl samples of the supernatant liquor from each well were removed and tested for antigen P24, which is produced by the human immunodeficiency virus using the standard Coulter Antigen (P24) ELISA test. The presence of antigen P24 thus indicates the presence of HIV.

The test described above gives an indication both of the test compound's inhibitory effect on virus release, and of the test compound's inhibitory effect on virus attachment and penetration.

Test B

Mammalian lymphocyte (H9IIIB) cells which were chronically infected with HIV were diluted to a concentration of $2x10^5 ml^{-1}$ in a culture medium (RPMI 1640). 1.0ml aliquots of this solution were placed in the wells of a tissue culture plate together with 1.0ml aliquots of an aqueous solution of the compound under test. A range of initial concentrations of test compound solution were used (0.2, 2, 20 and $200mgml^{-1}$), thus giving test compound concentrations of 0.1, 1, 10 and $100mgml^{-1}$ in the wells.

The infected cell/test compound mixtures were then incubated at 37°C.

On following days, for example day 1 and day 4, 200µl samples of the supernatant liquor from each well were removed and tested for antigen P24, which is produced by the human immunodeficiency virus. The presence of antigen P24 thus indicates the presence of HIV.

The test described above gives an indication of the test compound's inhibitory effect on virus release.

## Claims

1. A process for the manufacture of a pharmaceutical composition which comprises mixing a compound of formula I,

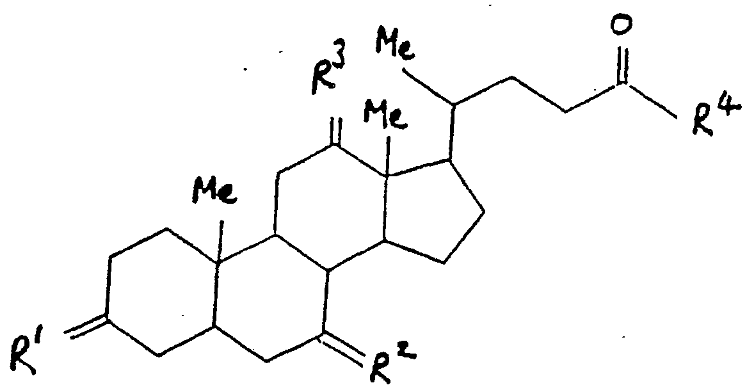

I

wherein
$R^1$ and $R^2$ both represent (H,α-OH) or both represent O;
$R^3$ represents (H,H) or O; and
$R^4$ represents OH or $NH(CH_2)_2SO_3H$;
and pharmaceutically acceptable salts thereof;
provided that
i) when $R^1$ and $R^2$ both represent (H,α-OH) and $R^3$ represents (H,H), then $R^4$ represents $NH(CH_2)_2SO_3H$; and
ii) when $R^1$ and $R^2$ both represent O and $R^3$ represents O, then $R^4$ represents $NH(CH_2)_2SO_3H$;
with a pharmaceutically acceptable adjuvant, diluent or carrier.

2. The use of a compound of formula I as defined in claim 1, but without proviso (ii), as active ingredient in the manufacture of a medicament for the treatment of diseases caused by an enveloped virus.

3. The use according to claim 2, wherein the disease is AIDS.

4. The use according to claim 2, wherein the disease is influenza or parainfluenza.

5. The use according to claim 2, wherein the disease is herpes.

6. The use according to any one of claims 2 to 5, wherein the compound of formula I is dehydrocholic acid.

7. The use according to any one of claims 2 to 5, wherein the compound of formula I is taurochenodeoxycholic acid.

8. A method of treatment or prophylaxis of a disease caused by an enveloped virus, which comprises administration of a therapeutically effective amount of a compound of formula I as defined in claim 2 to a patient suffering from or at risk of contracting such a disease.